# EUROPEAN PATENT SPECIFICATION

(11) **EP 1 424 084 B1**
(45) Date of publication and mention of the grant of the patent: **29.07.2009**
(21) Application number: 03026743.9
(22) Date of filing: 21.11.2003
(51) Int. Cl.: A61L 2/07

(54) **Device and process for steam sterilisation**
Vorrichtung und Verfahren zur Dampfsterilisation
Dispositif et procédé pour stérilisation à vapeur

(30) Priority: 26.11.2002 IT MI20022505
(43) Date of publication of application: 02.06.2004
(73) Proprietor: M.O.COM. S.r.L., 20124 Milano (IT)
(72) Inventor: Piazzalunga, Gianfranco, 20080 Casarile (IT); Tosi, Daniele Marino, 27018 Vidigulfo (Pavia) (IT)
(74) Representative: Lunati, Vittoriano

(56) References cited:
- EP-A- 0 937 465
- EP-A- 0 992 247
- DE-C- 19 930 546
- PATENT ABSTRACTS OF JAPAN vol. 1996, no. 03, 29 March 1996 (1996-03-29) & JP 07 299125 A (YOSHIDA DENTAL MFG CO LTD), 14 November 1995 (1995-11-14)

## Description

The invention relates to a device for steam sterilisation, in particular for sterilisation in the dental field.

It is known that steam sterilizers, in particular those intended for sterilisation of dental instruments, substantially comprise an autoclave housed in a box-shaped container and defining a sterilisation chamber accessible through a front door that can be opened and in which the items to be sterilised are placed:

Also disposed in the sterilizer container is a feed tank holding distilled water, which is connected to the sterilisation chamber through a feeding duct provided with a pump. Through one or more electric resistances the water fed to the sterilisation chamber is heated to form steam to a temperature and pressure adapted to perform a sterilising action on the items arranged in the chamber. The sterilisation chamber is further connected with an exhaust tank through a first exhaust duct, in which a heat exchanger is provided which comprises a cooling pipe coil, generally set in the feed tank under the level of the supplied water. This first duct is used to evacuate the sterilisation chamber by discharging condensate and steam from the chamber under pressure.

Also provided between the sterilisation chamber and exhaust tank is a second exhaust duct comprising a heat exchanger and a suction pump downstream of the exchanger. In particular the heat exchanger is of a type provided with a pipe coil air-cooled through fans for example.

This second duct is used to complete evacuation of the chamber and create a vacuum in the chamber before the true sterilisation. Since the boiling condensate discharged from the sterilisation chamber is not passed through the second exhaust duct, the amount of condensation water to be conveyed by the pump can be greatly reduced and therefore it is possible to use a less expensive pump, in particular a diaphragm pump.

The sterilisation cycle is performed in an automatic manner by operating, in a pre-established sequence, the pumps and a plurality of valves disposed in the different ducts.

These known sterilizers are somewhat complicated as regards construction, in particular due to arrangement of the pipe coil in the feed tank. In fact, these tanks are made of plastic material by a blowing process and a subsequent working is required to enable fastening of the pipe coil therein and connection to the pipe fittings of the exhaust duct. This involves an increase in costs.

In addition the exhaust tank must have a relatively high capacity and therefore must be disposed externally of the sterilizer, since there is no room enough inside. This constitutes an inconvenience above all for the user, who is obliged to find room for the tank and to accept an apparatus of greater bulkiness. Arrangement of the exhaust tank internally of the sterilizer container would at least require the tank volume to be greatly reduced and would involve an undesirable temperature increase in the regions surrounding the tank, making the operating conditions of the adjacent elements or devices heavier.

Are known sterilizers in which the heat exchanger and the water feed tank are separated, for example two of these sterilizer patent applications EP-A-937465 and EP-A-0992247. Nevertheless these sterilizers comprehend bulkiness heat exchanger comprehending fans or like, so these sterilizers have the same drawbacks.

Under this situation, the present invention mainly aims at providing a device and a process for steam sterilisation, in particular for sterilisation in the dental field, capable of overcoming the above inconveniences and problems.

It is an important aim of the invention to provide a sterilisation device and process enabling the exhaust tank to be disposed within the sterilizer, without this involving an increase in the sizes of the sterilizer container as well as temperature increases that are harmful to a good operation of the apparatuses located inside the sterilizer.

It is another aim of the invention to provide a device and a process of the above kind, enabling the sterilizer structure to be simplified, while at the same time reducing manufacturing costs and time of same.

It is a further aim of the invention to provide a sterilisation device and a process that do not involve important increases in the duration of the sterilisation cycle as compared with the duration of the usual cycles of known sterilizers.

A still further aim of the invention is to provide a sterilisation device and process which do not require special machining operations to be carried out on the feed tank for rendering it operative within the sterilizer.

The foregoing and further aims that will become more apparent in the following are achieved by a device and a process for steam sterilisation, in particular for sterilisation in the dental field, having the features set out in claims 1 and 11, respectively. Preferred embodiments of the invention are specified in the other claims.

Further details and advantages of the invention will be best understood from the following detailed description of a preferred embodiment of the invention, illustrated by way of non-limiting example in the accompanying drawings, in which:
- Fig. 1 is a diagram of a sterilisation device in accordance with the invention;
- Fig. 2 is a perspective view of the arrangement of the exhaust tank within the sterilizer container;
- Fig. 3 is a graph of the pressure course in the sterilisation chamber during a sterilisation cycle of the fractional vacuum type in accordance with the invention;
- Figs. 4, 5, 6, 7, 8 and 9 show hydraulic diagrams of the device in accordance with the invention where the fluid paths of travel during some steps of the sterilisation cycle in accordance with the invention are each time highlighted.

With reference to the drawings, a device 1 for steam sterilisation in accordance with the invention comprises, in a manner known by itself, an autoclave 2 of substantially cylindrical shape, internally defining a sterilisation chamber 3 adapted to house the items to be sterilised, in particular instruments or implements for dental use. Associated with the autoclave 2 is a steam generator 2a of a type known by itsetf.

The sterilisation chamber 3 is fed with water from a feed tank 4 through a feeding duct 5, in which a feed pump 6, a check valve 7 and an on-off valve 8 are disposed.

A first outlet duct 9 in which an on-off valve 10 is disposed comes out of the bottom of chamber 3. A second outlet duct 11, provided with an on-off valve 12 too, comes out of the upper part of chamber 3.

Downstream of valves 10 and 12 ducts 9 and 11 are connected together to a common exhaust duct 13, terminating in an exhaust tank 14 and passing through a heat exchanger 15, in particular an air-cooled heat exchanger of known type comprising a cooling pipe coil 15a cooled by fans not shown, for example.

A three-way valve 16 is disposed in duct 13, downstream of the heat exchanger 15, which valve is adapted to bring duct 13 into communication with the exhaust tank 14 selectively through a direct branch 13a or a branch 13b in which a suction pump 17 is positioned, a diaphragm vacuum pump of known type, for example.

Upstream of exchanger 15 and of the connecting points of the two outlet ducts 9 and 11, the exhaust duct 13 is connected with a first air-feeding duct 18, provided with an on-off valve 19. A second air-feeding duct 20, in which a filter 21, an on-off valve 22 and a check valve 23 are disposed, enters the sterilisation chamber 3 at the upper part thereof.

As can be viewed from the above description, a device 1 in accordance with the invention comprises an exhaust circuit with a single exhaust duct 13 in which the heat exchanger is located and through which both steam and boiling condensate as well as air discharged from the sterilisation chamber during the sterilisation cycle are caused to pass. The exhaust duct that in the known art passes through the feed tank, in which it has the form of a cooling pipe coil, is no longer present. The heat exchanger 15 is in fact separated from the feed tank 4.

In addition, the sterilisation chamber 3 is connected with such a single exhaust duct 13 both at the lower part of the chamber and at the upper part thereof.

In the sterilisation device 1 in accordance with the invention therefore no problems connected with the machining of the feed tank, installation of the cooling pipe coil within the feed tank the connection of said coil with the pipe fittings of the exhaust duct exist any longer.

The capacity being the same, the feed tank 4 can be less bulky so that it is possible to save room within the container 24 of the sterilizer. Likewise the presence of a single exhaust duct 13 enables further room to be saved internally of the sterilizer container 24.

Advantageously, it is provided that this available room be used for arranging the exhaust tank 14 internally of the sterilizer container 24.

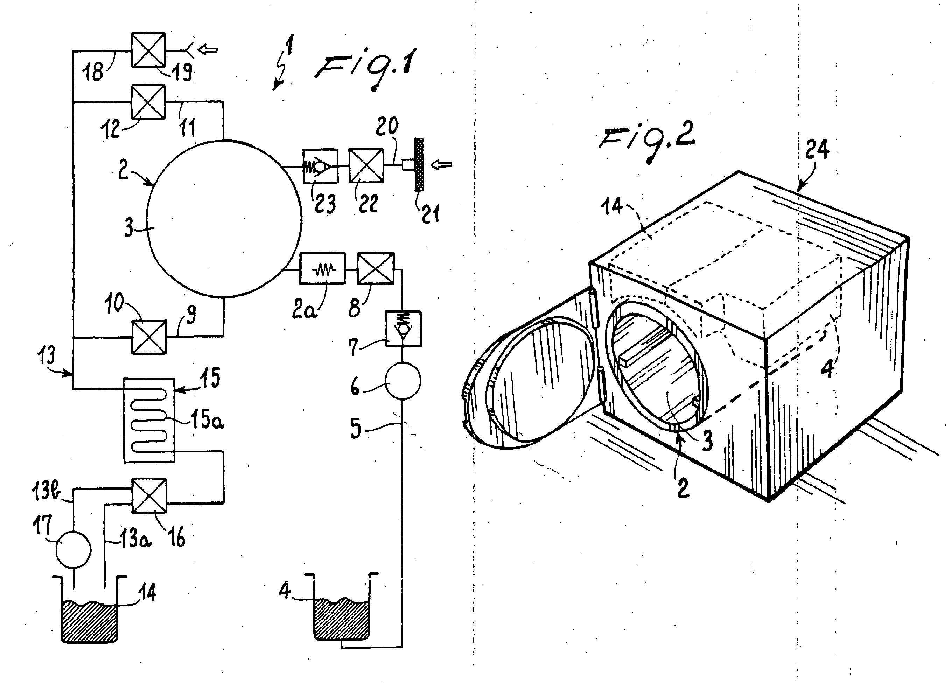

A possible arrangement of this tank is shown in Fig. 2. As can be seen, tank 14 can take up the upper part of the sterilizer, being such shaped that the lower part thereof substantially has the conformation of a cylindrical sector substantially matching a corresponding upper portion of the cylindrical surface of the autoclave 2, and the side and upper parts substantially have a parallelepiped conformation to adapt themselves to the volume defined by the side and upper walls of the box-shaped sterilizer container 24.

Advantageously, within the space above the autoclave 2 it is possible to combine both the exhaust tank 14 and the feed tank 4, making them of one piece construction, but hydraulically separated. Since there are no cooling pipe coils in either of the two tanks, particular machining operations are not required and installation of said tanks is greatly simplified as it substantially only involves connection of the feeding duct 5 and branches 13a and 13b to the respective tanks 4 and 14.

Arrangement of the exhaust tank 14 within the sterilizer container 24 may involve some reduction of the tank capacity, which will bring about a reduced heat-dissipation capability of the collected water and higher heating of the parts surrounding the tank within the sterilizer.

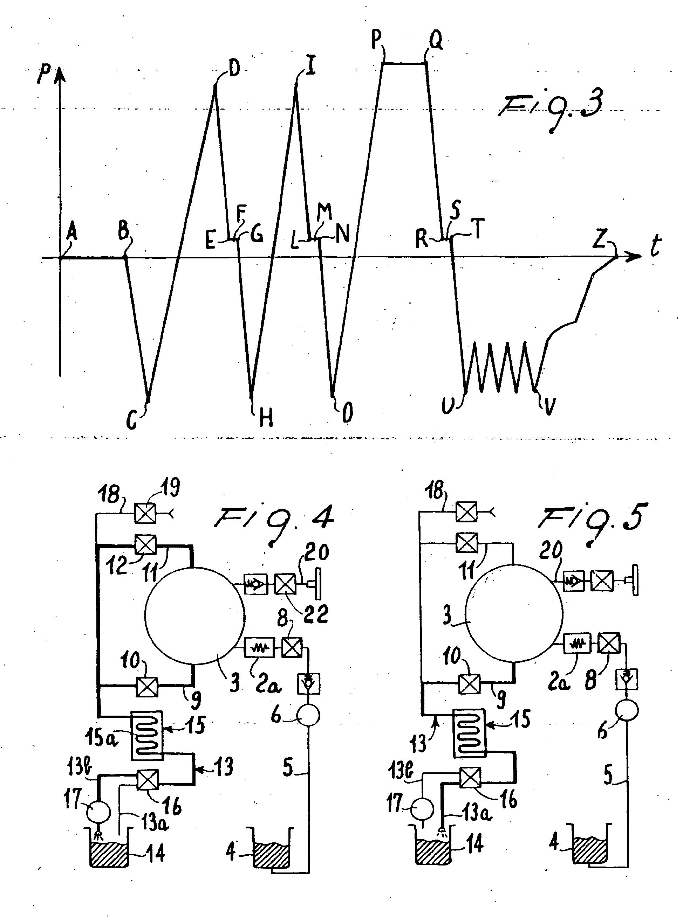

Advantageously, in accordance with an aspect of the invention, this occurrence can be obviated by a sterilisation process that differs from known processes and that will be described based on the graph in Fig. 3 and the diagrams in Figs. 4-9. Shown in the graph is the course of pressure *p* in the sterilisation chamber 3 versus time *t*.

After an initial step that may comprise an initial heating of the sterilisation chamber 3 (segment A-B of the graph in Fig. 3), the process involves a step of creating a pre-vacuum in the sterilisation chamber 3, which step is indicated by segment B-C in the graph in Fig. 3 and is shown in the diagram in Fig. 4.

During this step the on-off valves 8, 19 and 22 are closed, whereas valves 10 and 12 are open and valve 16 is in the position enabling communication of the exhaust duct 13 with the exhaust branch 13b. The vacuum pump 17 is in operation and it sucks air from the sterilisation chamber 3 through ducts 9, 11, 13 and 13b, as shown by the solid lines in bold type in Fig. 4. The air passes through the heat exchanger 15.

The vacuum in chamber 3 may reach -0.80 bar for example. During this step one or more short pulsed steam admissions to chamber 3 can be carried out through opening of valve 8 and closure of valve 12, to clean chamber 3 in a more complete manner.

When the desired vacuum has been reached, a pressurisation step of chamber 3 begins (segment C-D in the graph in Fig. 3) in a manner known by itself.

During this step valves 10, 12, 19, 22 are closed and valve 8 is open, whereas valve 16 is in a position connecting the exhaust duct 13 with the exhaust branch 13a. The feed pump 6 draws water from the feed tank 4 which water is heated in the steam generator 2a, reaching chamber 3 in the form of steam. Pressure in chamber 3 increases until 1.00 bar for example. Pump 17 is inoperative.

During this step, on reaching a pressure of 0.50 bar for example, a quick bleeding of air mixed with steam can be carried out from chamber 3 through the exhaust duct 13, by temporarily opening valve 10 or valve 12.

When the pre-established pressure has been reached, a step of evacuating condensate and steam from chamber 3 occurs (segment D-E in the graph in Fig. 3) by closing the feed valve 8 and opening the exhaust valve 10, whereas valve 16 is in a position causing operation of branch 13a of the exhaust duct 13. All the other valves are closed as shown in the diagram in Fig. 5 in which the solid lines in bold type highlight the fluid exhaust path from chamber 3.

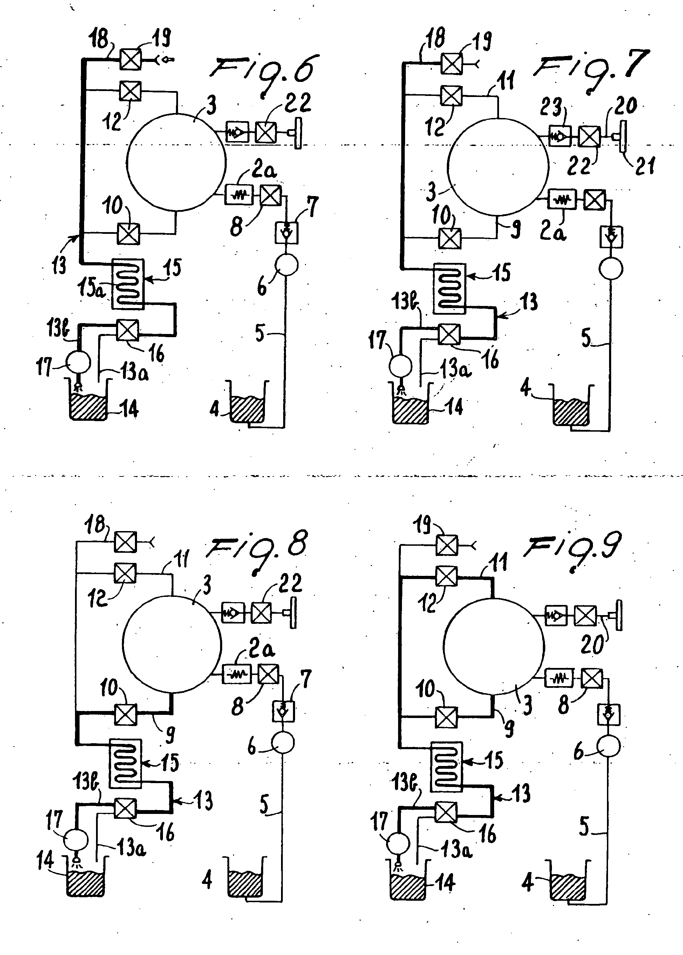

The exhausting operation takes place for example until a pressure of 0.10 bar is reached in chamber 3. At this point, in accordance with the invention, evacuation of the condensate and steam from the sterilisation chamber 3 is temporarily interrupted and a cooling step is carried out (segment E-F of the graph in Fig. 3), as shown in the diagram in Fig. 6, where the solid lines in bold type identify the operating ducts. Valves 8, 10, 12 and 22 are closed and valve 19 is open, whereas valve 16 is open towards branch 13b and the vacuum pump 17 is in operation. During this step pump 17 sucks air through ducts 18 and 13 and discharges it into the exhaust tank 14.

Advantageously the air in transit in duct 13 cools the exhaust duct and in particular the inside of the pipe coil 15a of heat exchanger 15. In addition it cleans the exhaust duct, in particular the inside of the pipe coil 15a of heat exchanger 15.

This step, that can be very short, is followed by an also short step of creating the vacuum in the exhaust duct 13, represented by segment F-G of the graph in Fig. 3, during which air suction from the outside is stopped by closure of valve 19, whereas pump 17 is still maintained in operation and creates the vacuum in the exhaust duct 13, and in particular in the pipe coil 15a of the heat exchanger, as viewed from the diagram in Fig. 7.

This step advantageously eliminates the residual condensate from the exhaust duct 13 and in particular from the heat exchanger 15, drying and cleaning the duct and bringing it to the optimal conditions for the subsequent step. It is to be noted that the sterilisation chamber 3 is not concerned with steps E-F-G and therefore pressure therein keeps constant during these steps.

At this point the residual evacuation of chamber 3 is carried out and the vacuum is created in said chamber (segment G-H of the graph in Fig. 3). First, valve 10 can be opened for evacuation of the liquid phase from chamber 3 through the outlet duct 9 (Fig. 8) and then valve 12 too is opened and the vacuum is created in chamber 3 through both ducts 9 and 11 (Fig. 9). Pressure in chamber 3 quickly decreases, until -0.80 bar for example.

Thanks to the preceding cooling and the preceding cleaning, drying and vacuum-creating step, the heat exchanger 15 enables quick condensing of the steam sucked from chamber 3 by pump 17 and quick evacuation, by the pump itself, of the formed condensate.

As is apparent from the graph in Fig. 3, either a step of pressurisation and subsequent evacuation and vacuum creation in chamber 3 can be repeated, these steps including the steps of cooling and creating the vacuum in the exhaust duct 13, in accordance with segments H-I-L-M-N-O, or chamber 3 can be brought to the sterilisation temperature and pressure, for carrying out the true sterilisation process. Pressurisation of chamber 3 takes place following segment O-P in the manner already described for segments C-D and H-I, whereas the sterilisation process takes place following segment P-Q in a manner known by itself, pre-established temperature and pressure conditions being automatically maintained in the sterilisation chamber 3.

When the sterilisation step has been completed, chamber 3 is evacuated by first carrying out steam and condensate evacuation from the chamber itself through the exhaust ducts 9 and 13 (segment Q-R of the graph in Fig. 3), and then executing the steps of cooling and creating the vacuum in duct 13 and heat exchanger 15 (segments R-S-T in the graph in Fig. 3) as already described for segments D-E-F-G and I-L-M-N in the graph in Fig. 3. Subsequently evacuation and vacuum creation in chamber 3 is completed (segment T-U in the graph in Fig. 3) as already explained for segments G-H and N-O of the same graph.

The sterilisation cycle may now contemplate a step of drying the sterilised items, through a succession of pulsed air admissions to chamber 3 from duct 20, by intermittently opening and closing valve 22 and keeping the vacuum pump 17 in operation, all that following a technique known by itself. Pressure in chamber 3 varies following the course highlighted between points U and V in the graph in Fig. 3.

Finally chamber 3 is gradually brought to the atmospheric pressure following segment V-Z in the graph in Fig. 3, by aeration of the chamber itself through duct 20 in a manner known by itself.

The described invention achieves important advantages.

Thanks to the constructional simplification of the hydraulic circuit of the sterilizer and the elimination of the cooling pipe coil from the feed tank, on the one hand it is possible to gain available room within the sterilizer container and dispose the whole exhaust duct as well as the exhaust tank therein, without increasing the sterilizer container sizes, and on the other hand it is possible to facilitate the sterilizer manufacture, since mounting of pipe coils within the tanks is not required.

The sterilizer having an internal instead of an external exhaust tank is also more practical for the user.

Insertion of the step of cooling and creating the vacuum in the exhaust duct during evacuation of steam and boiling condensate from the sterilisation chamber enables overheating of the heat exchanger of the exhaust circuit to be avoided and the efficiency thereof to be improved, and in addition it allows to prevent an undesirable heating from occurring in the region surrounding the exhaust tank, without on the other hand substantially increasing duration of the sterilisation cycle.

The arrangement of the two ducts 9 and 11 coming out from below and from top of chamber 3 respectively and opening into the same exhaust duct 13, and the possibility of selecting a direct evacuation route through valve 16 or an evacuation route through the suction pump 17 allows some freedom in selecting the exhaust path of the steam or the air/steam mixture in the developing step, so that the device better adapts itself to the particular requirements.

The hydraulic circuit can be conceived in such a manner that, in case of lack of electric energy, pressure in the autoclave automatically goes down to the level of the atmospheric pressure, making the rest position of the solenoid exhaust valves to correspond with the position enabling passage of fluid into the exhaust circuit.

The invention is susceptible of many modifications and variations in addition to those already described. Thus for example two heat exchangers could be provided in the exhaust duct 13, or several steps of cooling and cleaning the exhaust duct 13 and heat exchanger 15 could be provided when evacuation of the sterilisation chamber occurs, to a pressure between 0.50 and 0.00 bar for example. The exhaust tank 14 could also be placed, within the sterilizer, at a different position from the one herein depicted. Since the fluid reaching the exhaust tank 14 has been cooled by heat exchanger 15, the contents of tank 14 could be directly discharged into a centralised exhaust network, by establishing an external connection between the tank and the exhaust network itself, without interposition of pipe coils, tanks or other cooling means being required.

## Claims

1. A device for steam sterilisation, in particular for sterilisation in the dental field, comprising an autoclave (2) defining a sterilisation chamber (3) adapted to receive items to be sterilised, a water-feeding tank (4) connected with said chamber (3) through a feeding duct (5), an exhaust circuit (13, 9, 11) connected with said chamber (3) and provided with at least one heat exchanger (15), separated from said water-feeding tank (4), and at least one suction pump (17) for evacuation of said chamber (3), said exhaust circuit opening into an exhaust tank (14), **characterised in that** said exhaust circuit comprises a single exhaust duct (13) opening in said exhaust tank (14), said exhaust duct (13) being adapted to remove both condensate steam and air from said sterilization chamber (3).

2. A device as claimed in claim 1, **characterised in that** downstream of said heat exchanger (15) said exhaust duct (13) branches into a branch (13a) directly connected with said exhaust tank (14) and a branch (13b) connected with said exhaust tank (14) through said suction pump (17), and wherein a valve (16) is provided for bringing said exhaust duct (13) selectively into communication with either of said branches (13a, 13b).

3. A device as claimed in claim 1 or 2, **characterised in that** said exhaust circuit comprises two outlet ducts (9, 11) connected to said sterilisation chamber (3) and to said single exhaust duct (13), and **in that** said two outlet ducts (9, 11) come out of the bottom and the top of said chamber (3), respectively, each of said outlet ducts (9, 11) being provided with an on-off valve (10, 12).

4. A device as claimed in claim 3, **characterised in that** upstream of said heat exchanger (15) and of the connecting points of said outlet ducts (9, 11) said exhaust duct (13) is connected with an air-admitting duct (18).

5. A device as claimed in one or more of the preceding claims, **characterised in that** said exhaust tank (14) is disposed within a sterilisation container (24) housing the device.

6. A device as claimed in claim 5, **characterised in that** said exhaust tank (14) is made of one piece construction with said water-feeding tank (4), but is hydraulically separated therefrom.

7. A device as claimed in claim 5 or 6, **characterised in that** said autoclave (2) has a substantially semi-cylindrical shape and said exhaust tank (14) is disposed above said autoclave (2) and has a lower surface the shape of which substantially matches that of a corresponding upper portion of the cylindrical surface of said autoclave (2).

8. A device as claimed in one or more of the preceding claims, **characterised in that** said heat exchanger (15) is an air-cooled heat exchanger.

9. A device as claimed in one or more of the preceding claims, **characterised in that** said suction pump (17) is a diaphragm vacuum pump.

10. A steam sterilizer comprising a container (24) housing a sterilisation device (1) as claimed in one or more of claims 1 to 9, **characterised in that** the exhaust duct (13) and the exhaust tank (14) of the device (1) are entirely disposed internally of said container (24)

11. A process for steam sterilisation, in particular for sterilisation in the dental field, wherein it is provided a sterilisation chamber (3) adapted to receive items to be sterilised, and connected to a water-feeding tank (4) through a feeding duct (5), and an exhaust circuit (13, 9, 11) and provided with at least one heat exchanger (15), separated from said water-feeding tank (4), and at least one suction pump (17) for evacuation of said chamber (3), said exhaust circuit opening into an exhaust tank (14), **characterised in that** said sterilisation chamber (3) is brought to a sterilisation pressure and temperature and it is then evacuated by discharging condensate, steam and air from said chamber (3) through an a single exhaust duct (13) opening in said exhaust tank (14).

12. A process as claimed in claim 11, wherein evacuation of the condensate and steam from the sterilisation chamber (3) is temporarily interrupted, at least once, by a step of cooling the exhaust duct (13) and the heat exchanger (15).

13. A process as claimed in claim 12, wherein said cooling step is carried out by admission of air into the exhaust duct (13) and suction of the air by said suction pump (17).

14. A process as claimed in claim 12 or 13, wherein said cooling step first comprises an air-suction step through said exhaust duct (13) and said heat exchanger (15), and subsequently a step of creating vacuum in said exhaust duct (13) and said heat exchanger (15).

15. A process as claimed in one or more of claims 11 to 13, wherein said evacuation step is interrupted on reaching a gauge pressure of between 0.50 and 0.00 bar in said sterilisation chamber (3).

## Patentansprüche

1. Vorrichtung für die Dampfsterilisation, insbesondere für die Sterilisation im zahnärztlichen Bereich, bestehend aus einem Autoklav (2), der eine zum Enthalten von Sterilisationsgut geeignete Sterilisationskammer (3) bildet, einem Wasserspeisebehälter (4), der mit genannter Kammer (3) über einen Speisekanal (5) verbunden ist, einem Auslasskreis (13, 9, 11), der mit genannter Kammer (3) verbunden ist und mit mindestens einem von genanntem Speisebehälter (4) getrennten Wärmeaustauscher (15) und mit mindestens einer Saugpumpe (17) zum Entleeren genannter Kammer (3) ausgerüstet ist, wobei genannter Auslasskreis (13) in einen Auslassbehälter (14) ausläuft, **dadurch gekennzeichnet, dass** genannter Auslasskreis einen einzigen Auslasskanal (13) umfasst, der in den genannten Auslassbehälter (14) mündet, wobei genannter Auslasskanal (13) geeignet ist, sowohl das Kondenswasser als auch Dampf und Luft von genannter Sterilisationskammer (3) zu beseitigen.

2. Vorrichtung nach Anspruch 1, **dadurch gekennzeichnet, dass** sich nach genanntem Wärmeaustauscher (15) der genannte Auslasskanal (13) in einen direkt mit genanntem Auslassbehälter (14) verbundenen Zweig (13a) und einen mit genanntem Auslassbehälter (14) über die genannte Saugpumpe (17) verbundenen Zweig (13b) trennt und **dadurch**, dass ein geeignetes Ventil (16) vorgesehen ist, um genannten Auslasskanal (13) wahlweise mit einem oder dem anderen Zweig (13a, 13b) zu verbinden.

3. Vorrichtung nach Anspruch 1 oder 2, **dadurch gekennzeichnet, dass** genannter Auslasskanal zwei mit genannter Sterilisationskammer (3) und mit genanntem Auslasskanal (13) verbundene Ausgangskanäle (9, 11) umfasst und **dadurch**, dass sich die genannten zwei Ausgangskanäle (9, 11) vom Boden bzw. vom oberen Teil der genannten Sterilisationskammer (3) verzweigen, wobei jeder dieser Ausgangskanäle (9, 11) mit einem Sperrventil (10, 12) ausgerüstet ist.

4. Vorrichtung nach Anspruch 3, **dadurch gekennzeichnet, dass** vor genanntem Wärmeaustauscher (15) und den Verbindungsstellen genannter Ausgangskanäle (9, 11) der genannte Auslasskanal (13) mit einem Lufteinlasskanal (18) ausgerüstet ist.

5. Vorrichtung nach einem oder mehreren vorhergehenden Ansprüchen, **dadurch gekennzeichnet, dass** genannter Auslassbehälter (14) im Inneren eines Sterilisationsbehälters (24), der die Vorrichtung enthält, angeordnet ist.

6. Vorrichtung nach Anspruch 5, **dadurch gekennzeichnet, dass** genannter Auslassbehälter (14) aus einem Stück mit genanntem Wasserspeisebehälter (4) gebildet ist, von diesem aber hydraulisch getrennt ist.

7. Vorrichtung nach Anspruch 5 oder 6, **dadurch gekennzeichnet, dass** genannter Autoklav (2) eine im wesentlichen halbzylindrische Form aufweist und genannter Auslassbehälter (14) oberhalb genanntem Autoklav (2) angeordnet ist und eine untere Fläche aufweist, die im wesentlichen so geformt ist, dass sie in einen entsprechenden oberen Teil der zylindrischen Fläche des genannten Autoklav (2) eingreift.

8. Vorrichtung nach einem oder mehreren vorhergehenden Ansprüchen, **dadurch gekennzeichnet, dass** genannter Wärmeaustauscher (15) ein luftgekühlter Austauscher ist.

9. Vorrichtung nach einem oder mehreren vorhergehenden Ansprüchen,
**dadurch gekennzeichnet, dass** genannte Saugpumpe (17) als Membranvakuumpumpe ausgebildet ist.

10. Dampfsterilisator, bestehend aus einem Gehäusebehälter (24) einer Sterilisationsvorrichtung (1) nach einem oder mehreren vorhergehenden Ansprüchen 1 bis 9, **dadurch gekennzeichnet, dass** der Auslasskanal (13) und der Auslassbehälter (14) der Vorrichtung (1) im Inneren des genannten Behälters (24) angeordnet sind.

11. Verfahren für die Dampfsterilisation, insbesondere für die Sterilisation im zahnärztlichen Bereich, in dem eine Sterilisationskammer (3) vorgesehen ist, die geeignet zum Enthalten von Sterilisationsgut ist, die ferner mit einem Auslassbehälter (4) über einen Speisekanal (5) und einen Auslasskreis (13, 9, 11) verbunden und mit mindestens einem Wärmeaustauscher (15), der von genanntem Wasserauslassbehälter (4) getrennt ist, und mindestens einer Saugpumpe (17) für die Entleerung genannter Sterilisationskammer (3) ausgerüstet ist, wobei genannter Auslasskreis in genannten Auslassbehälter (14) mündet, **dadurch gekennzeichnet, dass** genannte Sterilisationskammer (3) auf Sterilisationsdruck und - Temperatur gebracht und dann entleert wird durch den Auslass von Kondenswasser, Dampf und Luft aus genannter Kammer (3) über einen einzigen Auslasskanal (13), der in genannten Auslassbehälter (14) mündet.

12. Verfahren nach Anspruch 11, in dem die Entleerung des Kondenswassers und des Dampfes von der Sterilisationskammer (3) zeitweilig mindestens einmal durch eine Kühlungsphase des Auslasskanals (13) und des Wärmeaustauschers (15) unterbrochen wird.

13. Verfahren nach Anspruch 12, in dem die genannte Kühlungsphase durch den Lufteinlass in den Auslasskanal (13) und das Ansaugen der Luft durch genannte Saugpumpe (17) erfolgt.

14. Verfahren nach Anspruch 12 oder 13, in dem genannte Kühlungsphase zuerst eine Lufteinlassphase durch genannten Auslasskanal (13) und genannten Wärmeaustauscher (15) und anschliessend eine Unterdruckphase des genannten Auslasskanals (13) und genannten Wärmeaustauschers (15) umfasst.

15. Verfahren nach einem oder mehreren Ansprüchen von 11 bis 13, in dem genannte Entleerungsphase beim Erreichen eines gemessenen Druckes zwischen 0,50 und 0,00 bar in genannter Sterilisationskammer (3) unterbrochen wird.

## Revendications

1. Dispositif pour la stérilisation à vapeur, en particulier pour la stérilisation dans le domaine odontologique, comprenant un autoclave (2) définissant une chambre de stérilisation (3) pouvant recevoir les objets à stériliser, un réservoir d'alimentation en eau (4) relié à ladite chambre (3) par un conduit d'alimentation (5), un circuit de vidange (13, 9, 11) relié à ladite chambre (3) et doté d'au moins un échangeur de chaleur (15), séparé dudit réservoir d'alimentation en eau (4), et d'au moins une pompe d'aspiration (17) pour l'évacuation de ladite chambre (3), ledit circuit de vidange (13) se terminant dans un réservoir de vidange (14), **caractérisé par le fait que** ledit circuit de vidange comprend un conduit unique de vidange (13) débouchant dans ledit réservoir de vidange (14), ledit conduit de vidange (13) pouvant éliminer la condensation, la vapeur et l'air de ladite chambre de stérilisation (3).

2. Dispositif selon la revendication 1, **caractérisé par le fait qu'**en aval dudit échangeur de chaleur (15) ledit conduit de vidange (13) se divise en un tronçon (13a) relié directement audit réservoir de vidange (14) et en un tronçon (13b) relié audit réservoir de vidange (14) par l'intermédiaire de ladite pompe d'aspiration (17), et **par le fait qu'**il prévoit une vanne (16) pouvant mettre en communication ledit conduit de vidange (13) sélectivement avec l'un ou l'autre des tronçons (13a, 13b).

3. Dispositif selon la revendication 1 ou 2, **caractérisé par le fait que** ledit conduit de vidange comprend deux conduits de sortie (9, 11) reliés à ladite chambre de stérilisation (3) et audit conduit de vidange (13), et **par le fait que** lesdits deux conduits de sortie (9, 11) s'éloignent respectivement du fond et de la partie supérieure de ladite chambre (3), chacun des conduits de sortie (9, 11) étant doté d'un robinet d'arrêt (10, 12).

4. Dispositif selon la revendication 3, **caractérisé par le fait qu'**en amont dudit échangeur de chaleur (15) et des points de raccord desdits conduits de sortie (9, 11) ledit conduit de vidange (13) est relié à un conduit d'arrivée d'air (18).

5. Dispositif selon une ou plusieurs des revendications précédentes, **caractérisé par le fait que** ledit réservoir de vidange (14) est placé à l'intérieur d'un récipient de stérilisation (24) contenant le dispositif.

6. Dispositif selon la revendication 5, **caractérisé par le fait que** la structure dudit réservoir de vidange (14) est réalisée d'une seule pièce avec ledit réservoir d'alimentation en eau (4), mais séparée hydrauliquement de ce dernier.

7. Dispositif selon la revendication 5 ou 6, **caractérisé par le fait que** ledit autoclave (2) possède une forme substantiellement semi-cylindrique et ledit réservoir de vidange (14) est placé au-dessus dudit autoclave (2) et possède une surface inférieure profilée de manière à épouser substantiellement une portion correspondante de la surface cylindrique dudit autoclave (2).

8. Dispositif selon une ou plusieurs des revendications précédentes, **caractérisé par le fait que** ledit échangeur de chaleur (15) est un échangeur refroidi à air.

9. Dispositif selon une ou plusieurs des revendications précédentes, **caractérisé par le fait que** ladite pompe d'aspiration (17) est une pompe à vide à membrane.

10. Stérilisateur à vapeur comprenant un récipient de logement (24) d'un dispositif de stérilisation (1) selon un ou plusieurs des revendications de 1 à 9,
**caractérisé par le fait que** le conduit de vidange (13) et le réservoir de vidange (14) du dispositif (1) sont entièrement placés à l'intérieur dudit récipient (24).

11. Procédé de stérilisation à vapeur, en particulier pour la stérilisation dans le domaine odontologique, dans lequel est prévue une chambre de stérilisation (3) pouvant recevoir les objets à stériliser, reliée à un réservoir de vidange d'eau (4) par l'intermédiaire d'un conduit d'alimentation (5), et à un circuit de vidange (13, 9, 11), et dotée d'au moins un échangeur de chaleur (15), séparé dudit réservoir de vidange d'eau (4), et d'au moins une pompe d'aspiration (17) pour l'évacuation de ladite chambre de stérilisation (3), ledit circuit de vidange débouchant dans ledit réservoir de vidange (14), **caractérisé par le fait que** ladite chambre de stérilisation (3) est portée à des pressions et des températures de stérilisation et qu'elle est ensuite évacuée en vidangeant la condensation, la vapeur et l'air de ladite chambre (3), à travers un seul conduit de vidange (13) débouchant dans ledit réservoir de vidange (14).

12. Procédé selon la revendication 11, dans lequel l'évacuation de la condensation et de la vapeur de la chambre de stérilisation (3) est interrompue temporairement, au moins une fois, par une phase de refroidissement du conduit de vidange (13) et de l'échangeur de chaleur (15).

13. Procédé selon la revendication 12, dans lequel ladite phase de refroidissement est effectuée par l'introduction d'air dans le conduit de vidange (13) et par l'aspiration de l'air par ladite pompe d'aspiration (17).

14. Procédé selon la revendication 12 ou 13, dans lequel ladite phase de refroidissement comprend d'abord une phase d'aspiration de l'air par ledit conduit de vidange (13) et ledit échangeur de chaleur (15), puis une phase de mise en dépression dudit conduit de vidange (13) et dudit échangeur de chaleur (15).

15. Procédé selon une ou plusieurs des revendications de 11 à 13, dans lequel ladite phase d'évacuation est interrompue une fois atteinte une pression mesurée comprise entre 0,50 et 0,00 bar dans ladite chambre de stérilisation (3).
